# EUROPEAN PATENT SPECIFICATION

(11) **EP 4 539 909 B1**
(45) Date of publication and mention of the grant of the patent: **28.10.2026**
(21) Application number: 23741555.9
(22) Date of filing: 16.06.2023
(51) Int. Cl.: A61M 5/24, A61M 5/145, A61M 5/28, A61M 5/315, A61M 5/31

(54) **PLUNGER ROD AND PLUNGER ROD ADAPTER FOR PISTONS**
KOLBENSTANGE UND KOLBENSTANGENADAPTER FÜR KOLBEN
TIGE DE POUSSÉE ET ADAPTATEUR DE TIGE DE POUSSÉE POUR PISTONS

(30) Priority: 16.06.2022 US 202263352637 P; 22.09.2022 US 202263376611 P
(43) Date of publication of application: 23.04.2025
(73) Proprietor: West Pharmaceutical Services, Inc., Exton, PA 19341 (US)
(72) Inventor: ANDREJKO, Brett, Exton, Pennsylvania 19341 (US); BANTZ, Daniel, Exton, Pennsylvania 19341 (US); SCHLEPPY, William, Exton, Pennsylvania 19341 (US); ALLARD, Bryan, Exton, Pennsylvania 19341 (US)
(74) Representative: Petraz, Gilberto Luigi
(86) International application number: PCT/US2023/068561
(87) International publication number: WO 2023/245154

(56) References cited:
- DE-C- 835 780
- US-A1- 2018 147 361
- US-A1- 2021 128 840
- US-A1- 2021 402 097

## Description

### CROSS REFERENCE TO RELATED APPLICATIONS

This application claims the benefit of U.S. Provisional Patent App. No. 63/352,637, filed June 16, 2022, and U.S. Provisional Patent App. No. 63/376,611, filed September 22, 2022.

### TECHNICAL FIELD

This application generally relates to an injector for injecting a medicament and plunger rods and plunger rod adapters, particularly to plunger rods and plunger rod adapters that include geometries that are complimentary to geometries of the interior of an associated piston, but that do not removably connect to the associated piston.

### BACKGROUND

Autoinjectors can provide intuitive, nonintimidating self-injection solutions for patients or caregivers. Some autoinjectors can include a plunger rod that is compatible with existing pistons of the prefilled syringes, which can improve access to various medicaments stored in such prefilled syringes and can allow the prefilled syringes to be transported and/or stored without a plunger rod. But unlike manually driven plunger rods used with standard prefilled syringes, plunger rods of some existing autoinjectors do not removably connect with the pistons of the prefilled syringes. Plunger rods of autoinjectors do not removably connect with the pistons of prefilled syringes at least in part because removably connecting a plunger rod with a piston can complicate loading the prefilled syringe into the autoinjector. Although some existing autoinjectors are operable with existing pistons of prefilled syringes, undesirable leakage of medicaments can occur. Document US 2021/128840A1 discloses stretchable plunger assemblies. Document US2018/147361A1 discloses a syringe plunger, bung and syringe. Document DE835780C discloses a syringe rack for cylinder ampoule for immediate injection. Document US2021/402097A1 discloses an auto-injector with uniform pressure exertion of a primary container.

Accordingly, there exists a need for improved plunger rods and plunger rod adapters that are operable with pistons of prefilled syringes, but that do not removably connect to the pistons and that mitigate or eliminate leakage.

### SUMMARY

These needs are met, to a great extent, by an injector for injecting a medicament according to annexed independent claim 1. Other advantageous features are defined in the annexed dependent claims.

Various additional features and advantages of this invention will become apparent to those of ordinary skill in the art upon review of the following detailed description of the illustrative embodiments taken in conjunction with the accompanying drawings.

### BRIEF DESCRIPTION OF THE DRAWINGS

The following detailed description is better understood when read in conjunction with the appended drawings. For the purposes of illustration, examples are shown in the drawings; however, the subject matter is not limited to the specific elements and instrumentalities disclosed. In the drawings:
FIG. 1 shows a plunger rod in accordance with aspects of the disclosure;
FIG. 2 shows the plunger rod disposed within a syringe barrel and operatively connected to a piston;
FIG. 3 shows a cross-section view of the plunger rod, piston, and syringe barrel of FIG. 2;
FIG. 4 shows an alternative geometry for a plunger rod or plunger rod adapter in accordance with aspects of this disclosure;
FIG. 5 shows example break loose glide profile data using the plunger of FIG. 1;
FIG. 6 shows another plunger rod disposed within a syringe barrel and operatively connected to a piston;
FIG. 7 shows a cross-section view of the plunger rod, piston, and syringe barrel of FIG. 6;
FIG. 8 shows still another plunger rod disposed within a syringe barrel and operatively connected to a piston, and not according to the claimed invention;
FIG. 9 shows a cross-section view of the plunger rod, piston, and syringe barrel of FIG. 8;
FIG. 10 shows a plunger rod and a plunger rod adapter in accordance with aspects of this disclosure;
FIG. 11 shows an autoinjector including a plunger rod in accordance with aspects of this disclosure; and
FIG. 12 shows an exploded view of the autoinjector of FIG. 11.

### DETAILED DESCRIPTION OF ILLUSTRATIVE EMBODIMENTS

This disclosure is directed plunger rods and plunger rod adapters that can be operable with pistons used with prefilled syringes. Interiors of the pistons can include a connector, such as threads or a snap fitting. The plunger rods and plunger rod adapters of this disclosure include geometries that do not removably connect to an associated piston. This makes the plunger rods and plunger rod adapters suitable for use with autoinjectors that do not removably connect to pistons. The geometries of the plunger rods and plunger rod adapters are complimentary to the geometry of the interior of an associated piston to substantially fill portions of the interior without removably connecting to the piston. By substantially filling the interior of the piston, the plunger rods and plunger rod adapters can resist deformation forces applied to the piston during injection and thereby mitigate or eliminate leakage of medicament between the pistons and the barrel of the syringe. These geometries can provide multiple and targeted points of interaction between the plunger rods or plunger rod adapters and the associated piston and can remove free space within the associated piston to prevent distortion and resist compression of the piston. The multiple points of interaction can balance piston-plunger stretch and compression, which benefits leak performance and reduces uncontrolled deformation from over compression.

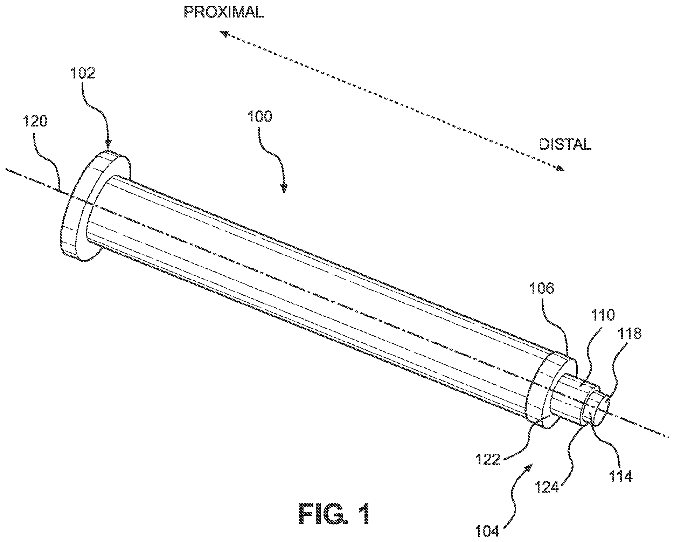

The plunger rods and plunger rod adapters of this disclosure can be self-aligning and can be used independent of elastomer composition of the pistons. The plunger rods and plunger rod adapters of this disclosure facilitate syringe operation at high delivery speed and/or high viscosity solutions where forces are significant. Additionally, the plunger rods and plunger rod adapters of this disclosure can provide a universal connection for different applications. For example, the plunger rods and plunger rod adapters of this disclosure can allow the same rod to be used in both a prefilled syringe system as well as a cartridge system. These and other aspects of the disclosure are described in further detail below in references to FIGS. 1-12.

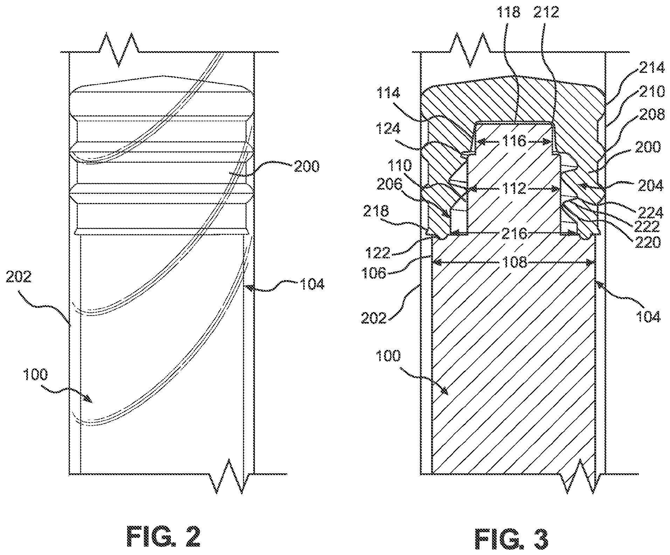

FIGS. 1-3 show views of a plunger rod 100 in accordance with aspects of this disclosure. FIG. 1 shows a perspective view of the plunger rod 100. FIG. 2 shows a partial view of the plunger rod 100 operatively connected to a piston 200 and disposed within the barrel 202 of a prefilled syringe. FIG. 3 shows a cross-section view of the plunger rod 100, piston 200, and barrel 202 of FIG. 2.

The plunger rod 100 can axially move the piston 200 within the barrel 202. The plunger rod 100 can include a proximal end 102 and a distal end 104. The distal end 104 can include a first portion 106 having a first diameter 108. The distal end 104 can include a second portion 110 distal to the first portion 106. The second portion 110 can have a second diameter 112 that is smaller than the first diameter 108. The second portion 110 can have an outer geometry that is complementary to an inner geometry of an interior 206 of the piston 200. The inner geometry of the piston can define a connector (e.g., threads, a snap fitting or the like) and the outer geometry of the distal end 104 can be complementary to the geometry of the connector. For example, the outer geometry can be complementary to a distal portion 212 of the interior 206 and/or can be complimentary to crests 220 of threads 204 of the interior 206. As a result of this configuration, when the plunger rod 100 is slid into the interior 206 of the piston 200, the first portion 106 can contact a proximal face 218 of the piston 200 and the second portion 110 can (e.g., simultaneously) contact the connector of the piston 200 (e.g., crests 220) without removably connecting to the piston 200. For example, the plunger rod 100 can be engaged with the piston 200 without removably connecting to the piston 200 such that the plunger rod 100 can disengage from the piston 200 without resistance from the piston 200 by sliding the plunger rod 100 proximally away from the piston 200. This can be advantageous for use with autoinjectors that do not removably connect with pistons of the syringes loaded in the autoinjectors. Because the outer geometry of the second portion 110 is complementary to the inner geometry of the interior 206 of the piston 200, the second portion 110 can substantially fill the interior 206 of the piston 200 to engage with the piston 200, which can allow the distal end 104 to resist deformation forces applied to the piston 200 during injections and thereby mitigate or eliminate leakage.

In embodiments such as shown in FIGS. 1-3, the plunger rod 100 can be operable with a piston 200 that includes threads 204 within the interior 206 and that includes a distal portion 212 that defines concavity extending distally beyond the threads 204. The distal end 104 can include the first portion 106 having a first diameter 108. The distal end 104 can include a second portion 110 that can be distal to the first portion 106. The second portion 110 can have a second diameter 112 that can be smaller than the first diameter 108. In embodiments, the distal end 104 can include a third portion 114 distal to the second portion 110. The third portion 114 can have a third diameter 116 that can be smaller than the second diameter 112. The third portion 114 can define a head 118. In embodiments, the head 118 can be substantially planar along a direction perpendicular to a longitudinal axis 120 of the plunger rod 100. Alternatively, the head 118 can define other shapes such as a cone shape, a dome shape, etc. The second portion 110 and the third portion 114 can together define an outer geometry that can substantially fill the interior 206 of the piston 200. By substantially filling the interior 206 of the piston 200, the distal end 104 can resist deformation forces applied to the piston 200 during injections and thereby mitigate or eliminate leakage. For example, the second diameter 112 can be dimensioned such that the second portion 110 abuts against at least one of the crests 220 of the threads 204 defined within the interior 206 of the piston 200 without contacting the flanks 222 or the roots 224 of the threads 204. In embodiments, the second portion 110 abuts against each of the crests 220 without contacting any of the flanks 222 or the roots 224. By abutting against at least one of the crests 220 of the threads 204 without contacting the flanks 222 or the roots 224 of the threads 204, the second portion 110 can engage with the piston 200 to resist deformation of the piston 200 or collapse of the piston 200 into the interior 206 without removably connecting to the piston 200. This deformation resistance can help to maintain contact between circumferential ribs 208 of the piston 200 and an interior wall 210 of the barrel 202, which can mitigate and/or eliminate leakage of medicament proximally between the piston 200 and the barrel 202.

In embodiments, the third diameter 116 can be dimensioned such that the third portion 114 abuts against a distal portion 212 (e.g., an interior wall) of the piston 200. The distal portion 212 can be defined within the interior 206 of the piston 200 at a location distal to the threads 204. By abutting against the distal portion 212, the third portion 114 can resist deformation of the piston 200 or collapse of the piston 200 into the interior 206. This deformation resistance can help maintain contact between a head 214 of the piston 200 and the interior wall 210, which can mitigate and/or eliminate leakage of medicament proximally between the piston 200 and the barrel 202. Additionally, the third portion 114 can transfer displacement force exerted on the plunger rod 100 to the distal portion 212 of the piston 200 to axially move the piston 200 distally in the barrel 202.

In embodiments, the first diameter 108 can be greater than an opening diameter 216 of the interior 206 of the piston 200. As a result of this configuration, a distal face 122 of the first portion 106 can abut against a proximal face 218 of the piston 200. By abutting against the proximal face 218 the distal face 122 can resist deformation of the piston 200 or collapse of the piston 200 into the interior 206. Additionally, the distal face 122 can transfer displacement force exerted on the plunger rod 100 to the proximal face 218 to axially move the piston 200 distally in the barrel 202.

In embodiments, the second portion 110 can include a distal face 124. The distal face 124 can abut against the distal portion 212 of the piston 200 to resist deformation of the piston 200 or collapse of the piston 200 into the interior 206. Additionally, the distal face 124 can transfer displacement force exerted on the plunger rod 100 to the distal portion 212 of the piston 200 to axially move the piston 200 distally in the barrel 202.

In embodiments, the head 118 can abut against the distal portion 212 of the piston 200 to resist deformation of the piston 200 or collapse of the piston 200 into the interior 206. Additionally, the head 118 can transfer displacement force exerted on the plunger rod 100 to the distal portion 212 of the piston 200 to axially move the piston 200 distally in the barrel 202.

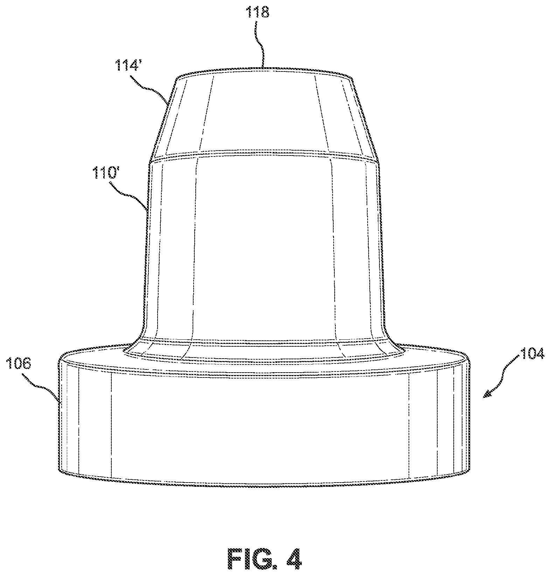

The first portion 106, second portion 110, and third portion 114 can be dimensioned a number of different ways to substantially fill the interior 206 of the piston 200 and thereby resist deformation of the piston 200. The first portion 106, second portion 110, and third portion 114 can be arranged concentrically along the longitudinal axis 120. In embodiments, the first portion 106 can be cylindrical. In embodiments, the second portion 110 can be cylindrical, as shown in FIGS. 1 and 3. In an alternative example, the second portion 110' can be conical with a distal taper, as shown in FIG. 4. In embodiments, the third portion 114 can be cylindrical, as shown in FIGS. 1 and 3. In an alternative example, the third portion 114' can be conical with a distal taper, as shown in FIG. 4. In embodiments, the second portion 110 can be conical with a distal taper and the third portion 114 can be cylindrical, or the second portion 110 can be cylindrical and the third portion 114 can be conical with a distal taper. Other permutations are possible.

In embodiments, the second portion 110 can have a length along the longitudinal axis 120 that is greater than a length along the longitudinal axis 120 of the third portion 114. Due to this configuration, the second portion 110 can substantially fill the interior 206 of the piston 200 that includes the threads 204 while the third portion 114 can substantially fill the interior 206 of the piston 200 disposed distally relative to the threads 204. In embodiments, the length of the second portion 110 can be at least twice the length of the third portion 114. Additionally or alternatively, the length along the longitudinal axis 120 of the second portion 110 can be greater than a length along the longitudinal axis 120 of the first portion 106. In embodiments, the distal end 104 can be integral with the plunger rod 100.

The plunger rod 100 was tested with a prefilled syringe containing a fluid having viscosity of 957 cP at a variety of different speeds of the plunger 100/piston 200 within the barrel 202. For each speed the pressure and force applied to the piston 200 was calculated and force measurements were taken. Table 1 below shows a summary of the results of these tests and demonstrates that the plunger rod 100 of this disclosure is suitable for leak-free use with a prefilled syringe under a variety of different conditions. Note: The viscosity is converted to SI units according to the following relationship: 1 cP = 1 mPa·s (millipascal-second). For the pressure, the following relationship applies: 1 PSI = 6894.76 Pa.

**Table 1**

| **Viscosity Measured (cP)** | **Speed (mm/min)** | **Calculated Pressure (PSI)** | **Calculated Force (N)** | **Measured Force (N)** | **Results: Leak (Y\N)** |
|---|---|---|---|---|---|
| 957.0 (+/-96) | 50 | 916 | 367 | 294 | N |
| 957.0 (+/-96) | 75 | 1364 | 550 | 449 | N |
| 957.0 (+/-96) | 100 | 1832 | 734 | 548 | N |
| 957.0 (+/-96) | 125 | 2290 | 917 | 607 | N |
| 957.0 (+/-96) | 150 | 2747 | 1100 | 703 | N |
| 957.0 (+/-96) | 175 | 3205 | 1284 | 772 | N |

The plunger rod 100 was also tested using a mechanical testing machine to measure break loose and extrusion force profiles for a variety of different speeds of the plunger 100/piston 200 within the barrel 202. The different speeds included speeds of 100 mm/min, 380 mm/min, and 450 mm/min. The plunger rod 100 was tested at least 10 times at each speed with different prefilled syringes containing different fluids at ambient temperature. The different fluids had respective viscosities of 1 cP, 20 cP, and 100 cP. During each test for all fluids and at all speeds tested, no leaks were detected when the plunger rod 100 was used with the piston 200. Table 2 below shows the test results for the plunger rod 100 under some of these test conditions as compared to two known plunger rods with different distal end geometries: Plunger Rod A and Plunger Rod B. Plunger Rod A had a distal end that fits within the interior 206, but that is smaller than the threads 204 so as to not contact the crests 220 of the threads 204. Moreover, no portion of the distal end of Plunger A contacts the proximal face 218 of the piston 200 when Plunger A moves the piston 200. Plunger Rod B has a distal end that occupies less than half of the interior 206 of the piston 200 and that does not contact any of the crests 220 of the threads 204 or the distal portion 212 of the interior 206 when Plunger B moves the piston 200. Plunger B includes a distal face that contacts the proximal face 218 of the piston 200 when Plunger A moves the piston 200. Table 2 shows that each of Plunger Rods A and B exhibited leaks under at least some of the conditions tested while plunger rod 100 did not leak under any of the test conditions.

**Table 2**

| **Viscosity and Speed (cP; mm/min)** | **Leaks Detected Over Number of Tests Performed** | | |
|---|---|---|---|
| | **Plunger Rod A** | **Plunger Rod B** | **Plunger Rod 100** |
| 1; 380 | 0/10 | 0/10 | 0/10 |
| 1; 450 | 0/10 | 0/10 | 0/10 |
| 20; 380 | **10/10** | 0/10 | 0/10 |
| 20; 450 | **10/10** | 0/10 | 0/10 |
| 100; 380 | **10/10** | 0/10 | 0/10 |
| 100; 450 | **10/10** | **2/10** | 0/10 |

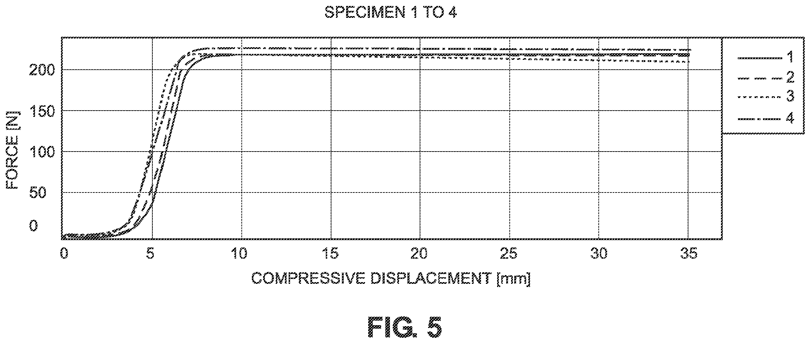

FIG. 5 shows example break loose glide profile data collected with the plunger rod 100, the piston 200, and a 100 cP fluid within the barrel 202. The plunger rod 100 moved the piston 200 at a speed of 380 mm/min and compression forces for motion during glide force profiles were collected. This data shows that the plunger rod 100 can create less distortion and compression when fluid is expelled from the barrel 202 even at relatively high speeds (e.g., 380 mm/min) and fluid viscosities (e.g., 100 cP). This data shows that the plunger rod 100 can mitigate or eliminate leakage.

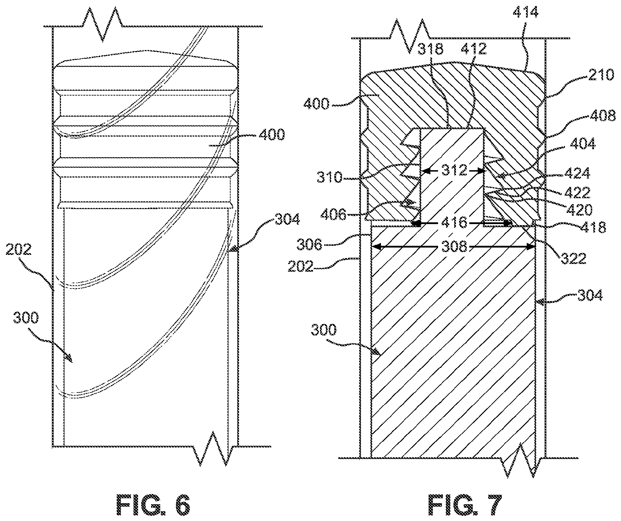

FIG. 6 shows a partial view of another plunger rod 300 operatively connected to another piston 400 and disposed within the barrel 202 of a prefilled syringe. FIG. 7 shows a cross-section view of the plunger rod 300, piston 400, and barrel 202 of FIG. 6. In embodiments such as shown in FIGS. 6 and 7, the plunger rod 300 can be operable with the piston 400 that includes threads 404 within the interior 406 of the piston 400 and that includes a distal portion 412 that defines a wall that can be perpendicular to a longitudinal axis of the piston 400 and that can be located a terminal distal end of the threads 404.

The plunger rod 300 and the piston 400 can include any of the respective features discussed previously in reference to the plunger rod 100 and the piston 200, where like parts correspond to like parts. The plunger rod 300 can differ from the plunger rod 100 in that the plunger rod 300 can have a distal end 304 with an outer geometry adapted to fit an inner geometry of the piston 400. For example, the distal end 304 of the plunger rod 300 can include a first portion 306 having a first diameter 308. The distal end 304 can include a second portion 310 that can be distal to the first portion 306. The second portion 310 can have a second diameter 312 that can be smaller than the first diameter 308. The second portion 310 can define a head 318. In embodiments, the head 318 can be substantially planar along a direction perpendicular to a longitudinal axis of the plunger rod 300. Alternatively, the head 318 can define other shapes such as a cone shape, a dome shape, etc. The second portion 310 can define an outer geometry that can substantially fill the interior 406 of the piston 400. By substantially filling the interior 406 of the piston 400, the distal end 304 can resist deformation forces applied to the piston 400 during injections and thereby mitigate or eliminate leakage. For example, the second diameter 312 can be dimensioned such that the second portion 310 abuts against at least one of the crests 420 of the threads 404 defined within the interior 406 of the piston 400 without contacting the flanks 422 or the roots 424 of the threads 404. In embodiments, the second portion 310 abuts against each of the crests 420 without contacting any of the flanks 422 or the roots 424. By abutting against at least one of the crests 420 of the threads 404 without contacting the flanks 422 or the roots 424 of the threads 404, the second portion 310 can engage with the piston 400 to resist deformation of the piston 400 or collapse of the piston 400 into the interior 406 without removably connecting to the piston 400. This deformation resistance can help to maintain contact between circumferential ribs 408 of the piston 400 and the interior wall 210 of the barrel 202, which can mitigate and/or eliminate leakage of medicament proximally between the piston 400 and the barrel 202.

In embodiments, the head 318 abuts against the distal portion 412 (e.g., an interior wall) of the piston 400. In embodiments, the distal portion 412 can be defined within the interior 406 of the piston 400 at distal terminal end of the threads 404. By abutting against the distal portion 412, the head 318 can resist deformation of the piston 400 or collapse of the piston 400 into the interior 406. This deformation resistance can help maintain contact between a head 414 of the piston 400 and the interior wall 210, which can mitigate and/or eliminate leakage of medicament proximally between the piston 400 and the barrel 202. Additionally, the head 318 can transfer displacement force exerted on the plunger rod 300 to the distal portion 412 of the piston 400 to axially move the piston 400 distally in the barrel 202.

In embodiments, the first diameter 308 can be greater than an opening diameter 416 of the interior 406 of the piston 400. As a result of this configuration, a distal face 322 of the first portion 306 can abut against a proximal face 418 of the piston 400. By abutting against the proximal face 418 the distal face 322 can resist deformation of the piston 400 or collapse of the piston 400 into the interior 406. Additionally, the distal face 322 can transfer displacement force exerted on the plunger rod 300 to the proximal face 418 to axially move the piston 400 distally in the barrel 202.

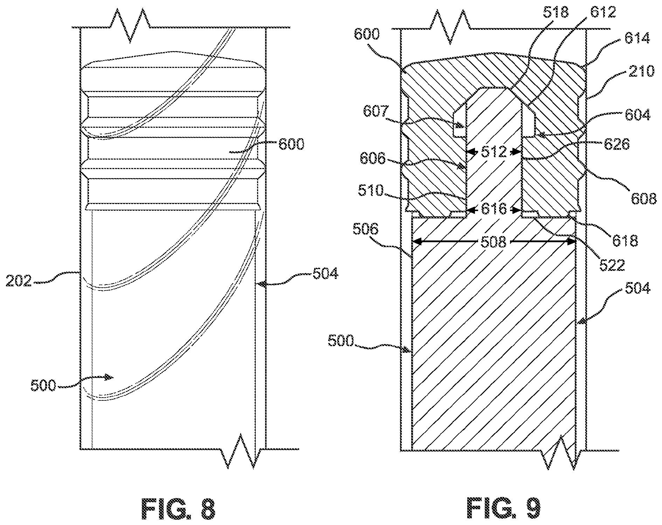

FIG. 8 shows a partial view of another plunger rod 500 operatively connected to another piston 600 and disposed within the barrel 202 of a prefilled syringe. This embodiment is not part of the claimed invention. FIG. 9 shows a cross-section view of the plunger rod 500, piston 600, and barrel 202 of FIG. 8. In embodiments such as shown in FIGS. 8 and 9, the plunger rod 500 can be operable with the piston 600, which includes a snap fitting 604 within the interior 606 of the piston 600.

The plunger rod 500 and the piston 600 can include any of the respective features discussed previously in reference to the plunger rod 100 and the piston 200, where like parts correspond to like parts. The plunger rod 500 can differ from the plunger rod 100 in that the plunger rod 500 can have a distal end 504 with an outer geometry adapted to fit an inner geometry of the piston 600. For example, the distal end 504 of the plunger rod 500 can include a first portion 506 having a first diameter 508. The distal end 504 can include a second portion 510 that can be distal to the first portion 506. The second portion 510 can have a second diameter 512 that can be smaller than the first diameter 508. The second portion 510 can define a head 518. In embodiments, the head 518 can be conical or dome shaped to correspond to the shape of the distal portion 612 of the piston 600. The second portion 510 can define an outer geometry that can substantially fill the interior 606 of the piston 600 except for regions 607 of the interior 606 that extend radially beyond an opening diameter 616 of the piston 600 and that define the snap fitting 604. For example, in embodiments the diameter of the second portion 510 is less than the opening diameter 616 of the piston such that second portion 510 does not connect with the snap fitting 604. By substantially filling the interior 606 of the piston 600 except for the regions 607 that define the snap fitting 604, the distal end 504 can resist deformation forces applied to the piston 600 during injections and thereby mitigate or eliminate leakage without removably connecting to the piston 600. For example, the second diameter 512 can be dimensioned such that the second portion 510 abuts against a circumferential inner wall 626 of the piston 600 but not against the snap fitting 604. That is, the second portion 510 does not engage with the snap fitting when the plunger rod 500 is slid within the interior 606 of the piston 600. In embodiments, the second portion 510 abuts against the entirety of the circumferential inner wall 626. By abutting against the circumferential inner wall 626 but not against the snap fitting 604, the second portion 510 can engage with the piston 600 to resist deformation of the piston 600 or collapse of the piston 600 into the interior 606 without removably connecting to the piston 600. This deformation resistance can help to maintain contact between circumferential ribs 608 of the piston 600 and the interior wall 210 of the barrel 202, which can mitigate and/or eliminate leakage of medicament proximally between the piston 600 and the barrel 202.

In embodiments, the head 518 abuts against a distal portion 612 (e.g., an interior wall) of the piston 600. By abutting against the distal portion 612, the head 518 can resist deformation of the piston 600 or collapse of the piston 600 into the interior 606. This deformation resistance can help maintain contact between a head 614 of the piston 600 and the interior wall 210, which can mitigate and/or eliminate leakage of medicament proximally between the piston 600 and the barrel 202. Additionally, the head 518 can transfer displacement force exerted on the plunger rod 500 to the distal portion 612 of the piston 600 to axially move the piston 600 distally in the barrel 202.

In embodiments, the first diameter 508 can be greater than the opening diameter 616 of the interior 606 of the piston 600. As a result of this configuration, a distal face 522 of the first portion 506 can abut against a proximal face 618 of the piston 600. By abutting against the proximal face 618 the distal face 522 can resist deformation of the piston 600 or collapse of the piston 600 into the interior 606. Additionally, the distal face 522 can transfer displacement force exerted on the plunger rod 500 to the proximal face 618 to axially move the piston 600 distally in the barrel 202.

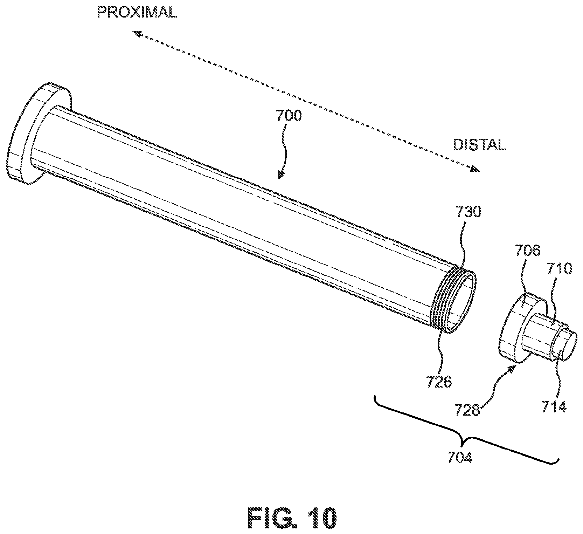

In embodiments such as shown in FIG. 10, the distal end 704 of the plunger rod 700 can define both a distal stem end 726 and a plunger rod adapter 728. The plunger rod adapter 728 can be removably connected to the distal stem end 726. The distal end 704 can include any of the features discussed above in reference to distal ends 104, 304, and 504 except that the plunger rod adapter 728 is removably connected to the distal stem end 726 instead of integrally formed therewith. For example, the plunger rod adapter 728 can include any of the geometries discussed previously in reference to the distal end 104. For example, the plunger rod adapter 728 can include a first portion 706, a second portion 710, and a third portion 714 that can respectively include any of the features discussed previously with respect to the first portions 106, 306, 506, the second portions 110, 110', 310, 510 and/or the third portions 114/114'.

The plunger rod adapter 728 can be removably connected to the distal stem end 726 via any number of connection techniques including adhering the plunger rod adapter 728 to the plunger rod 700 with an adhesive, fastening the plunger rod adapter 728 to the plunger rod 700 with a fastener, among other possibilities. For example, in embodiments the distal stem end 726 can include threads 730 and the plunger rod adapter 728 can be removably connected to the threads 730. The plunger rod adapter 728 can include internal threads that threadedly connect to the threads 730, or the plunger rod adapter 728 can be connected via other known connecting techniques such as snap fitting, adhering, etc. Because the plunger rod adapter 728 can include geometries for substantially filling the interior of the piston 200 without threadedly connecting to the piston 200, the plunger rod adapter 728 can adapt a plunger rod 700 with a threaded distal stem end 726 for use with, for example, autoinjectors that do not threadedly connect to the piston 200. That is, the plunger rod adapter 728 can be interposed between the threads 730 of the distal stem end 726 and the threads 204 of the piston 200 to connect the piston 200 with the plunger rod 700 without threadedly engaging the threads 204 of the piston 200.

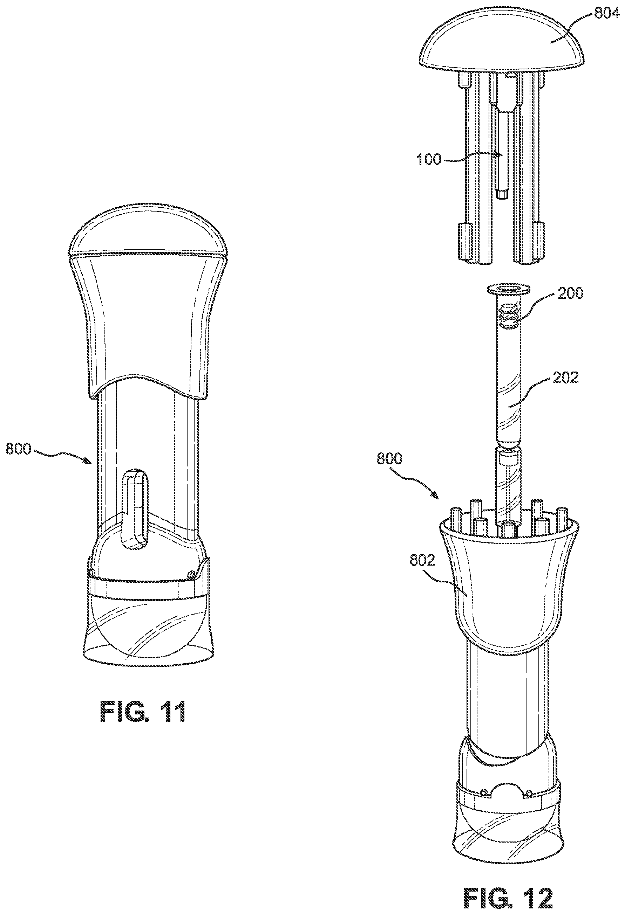

FIGs. 11 and 12 show views of an autoinjector 800 for injecting a medicament. The autoinjector 800 can include any of the plunger rods and/or plunger rod adapters described herein. For example, the autoinjector 800 can include the plunger rod 100. The autoinjector 800 can include a base 802 that can receive the barrel 202 containing the piston 200. The autoinjector 800 can further include a handle 804, which can be integrally or removably connected to the plunger rod 100. Depressing the handle 804 can move the plunger rod 100 and axially displace the piston 200 within the barrel 202 to inject the medicament into a patient.

It will be appreciated that the foregoing description provides examples of the disclosure. However, it is contemplated that other implementations of the invention may differ in detail from the foregoing examples. All references to the invention or examples thereof are intended to reference the particular example being discussed at that point and are not intended to imply any limitation as to the scope of the invention more generally. All language of distinction and disparagement with respect to certain features is intended to indicate a lack of preference for those features, but not to exclude such from the scope of the invention entirely unless otherwise indicated. All methods described herein can be performed in any suitable order unless otherwise indicated herein or otherwise clearly contradicted by context.

## Claims

1. An injector (800) for injecting a medicament, the injector (800) comprising:
a syringe barrel (202);
a piston (200, 400) movably disposed within the syringe barrel (202), the piston (200, 400) defining an interior (206, 406) extending from a proximal face (218, 418) of the piston (200, 400) to a distal portion (212, 412), the piston (200, 400) comprising threads (204, 404) comprising crests (220, 420);
a plunger rod (100, 300, 700) or a plunger rod adapter (728) for axially moving the piston (200, 400) within the syringe barrel (202), the plunger rod (100, 300, 700) or the plunger rod adapter (728) comprising:
a proximal end (102); and
a distal end (104, 304, 704) comprising:
a first portion (106, 306, 706) having a first diameter (108, 308, 708); and
a second portion (110, 110', 301, 710) distal to the first portion (106, 306, 706), the second portion (110, 110', 301, 710) having a second diameter (112, 312) smaller than the first diameter (108, 308, 708), wherein
the second portion (110, 110', 301, 710) has an outer geometry that is complementary to the distal portion (212, 412) of the interior (206, 406) of the piston (200, 400), and
the distal end (104, 304, 704) is configured such that when the plunger rod (100, 300, 700) or the plunger rod adapter (728) is slid into the interior of the piston (200, 400):
the first portion (106, 306, 706) contacts the proximal face of the piston (200, 400), and **characterized in that**
the second portion (110, 110', 301, 710) abuts against each of the crests (220, 420) of the threads (204, 404) of the interior (206, 406) of the piston (200, 400) without removably connecting to the piston (200, 400) such that the plunger rod (100, 300, 700) or the plunger rod adapter (728) is configured to disengage from the piston (200, 400) by sliding the plunger rod (100, 300, 700) or the plunger rod adapter (728) proximally away from the piston (200, 400).

2. The injector (800) of claim 1, wherein the outer geometry is configured to engage with the piston (200, 400) such that the second portion (110, 110', 301, 710) is configured to resist deformation forces applied to the piston (200, 400) during axial movement of the piston (200, 400) within the syringe barrel (202).

3. The injector (800) of claim 1, wherein the second portion (110, 110', 301, 710) is conical and distally tapers.

4. The injector (800) of claim 1, wherein the distal end further comprises a head that is configured to contact the distal portion (212, 412) of the interior (206, 406) of the piston (200, 400).

5. The injector (800) of claim 4, wherein the distal end (104, 304, 704) further comprises a third portion (114, 714) between the second portion (110, 110', 301, 710) and the head, the third portion (114, 714) has a third diameter (116) that is smaller than the second diameter (112, 312).

6. The injector (800) of claim 5, wherein the third portion (114, 714) is cylindrical or is conical and distally tapers.

7. The injector (800) of claim 5, wherein the first portion (106, 306, 706), the second portion (110, 110', 301, 710), and the third portion (114, 714) are concentrically arranged along a longitudinal axis of the plunger rod (100, 300, 700).

8. The injector (800) of claim 5, wherein a length of the second portion (110, 110', 301, 710) along a longitudinal axis is at least twice a length of the third portion (114, 714) along the longitudinal axis.

9. The injector (800) of claim 1, wherein the distal end (104, 304, 704) is integrally formed with the plunger rod (100, 300, 700) or the plunger rod adapter (728).

10. The injector (800) of claim 1, wherein:
the injector (800) comprises the plunger rod (100, 300, 700) and the plunger rod adapter (728), and
the distal end (104, 304, 704) is the plunger rod adapter (728) and is removably connected to the plunger rod (100, 300, 700).

## Patentansprüche

1. Injektor (800) zum Injizieren eines Arzneimittels, wobei der Injektor (800) umfasst:
einen Spritzenzylinder (202);
einen Kolben (200, 400), der innerhalb des Spritzenzylinders (202) beweglich angeordnet ist, wobei der Kolben (200, 400) einen Innenraum (206, 406) definiert, der sich von einer proximalen Fläche (218, 418) des Kolbens (200, 400) zu einem distalen Abschnitt (212, 412) erstreckt, wobei der Kolben (200, 400) Gewinde (204, 404) umfasst, die Scheitel (220, 420) umfassen;
eine Kolbenstange (100, 300, 700) oder einen Kolbenstangenadapter (728) zum axialen Bewegen des Kolbens (200, 400) innerhalb des Spritzenzylinders (202), wobei die Kolbenstange (100, 300, 700) oder der Kolbenstangenadapter (728) umfasst:
ein proximales Ende (102); und
ein distales Ende (104, 304, 704) umfassend:
einen ersten Abschnitt (106, 306, 706), der einen ersten Durchmesser (108, 308, 708) hat; und
einen zweiten Abschnitt (110, 110', 301, 710), der zum ersten Abschnitt (106, 306, 706) distal ist, wobei der zweite Abschnitt (110, 110', 301, 710) einen zweiten Durchmesser (112, 312) hat, der kleiner ist als der erste Durchmesser (108, 308, 708), worin
der zweite Abschnitt (110, 110', 301, 710) eine äußere Geometrie hat, die zum distalen Abschnitt (212, 412) des Innenraums (206, 406) des Kolbens (200, 400) komplementär ist, und
das distale Ende (104, 304, 704) derart ausgelegt ist, dass, wenn die Kolbenstange (100, 300, 700) oder der Kolbenstangenadapter (728) in den Innenraum des Kolbens (200, 400) geschoben wird:
der erste Abschnitt (106, 306, 706) die proximale Fläche des Kolbens (200, 400) berührt, und **dadurch gekennzeichnet, dass**
der zweite Abschnitt (110, 110', 301, 710) gegen jeden der Scheitel (220, 420) der Gewinde (204, 404) des Innenraums (206, 406) des Kolbens (200, 400) stößt, ohne sich abnehmbar mit dem Kolben (200, 400) zu verbinden, so dass die Kolbenstange (100, 300, 700) oder der Kolbenstangenadapter (728) dafür ausgelegt ist, sich vom Kolben (200, 400) dadurch zu lösen, dass die Kolbenstange (100, 300, 700) oder der Kolbenstangenadapter (728) proximal weg vom Kolben (200, 400) geschoben wird.

2. Injektor (800) nach Anspruch 1, worin die äußere Geometrie dafür ausgelegt ist, in den Kolben (200, 400) derart in Eingriff zu gehen, dass der zweite Abschnitt (110, 110', 301, 710) dafür ausgelegt ist, Verformungskräften zu widerstehen, die auf den Kolben (200, 400) während der axialen Bewegung des Kolbens (200, 400) innerhalb des Spritzenzylinders (202) aufgebracht werden.

3. Injektor (800) nach Anspruch 1, worin der zweite Abschnitt (110, 110', 301, 710) konisch ist und sich distal verjüngt.

4. Injektor (800) nach Anspruch 1, worin das distale Ende ferner einen Kopf umfasst, der dafür ausgelegt ist, den distalen Abschnitt (212, 412) des Innenraums (206, 406) des Kolbens (200, 400) zu berühren.

5. Injektor (800) nach Anspruch 4, worin das distale Ende (104, 304, 704) ferner einen dritten Abschnitt (114, 714) zwischen dem zweiten Abschnitt (110, 110', 301, 710) und dem Kopf umfasst, wobei der dritte Abschnitt (114, 714) einen dritten Durchmesser (116) hat, der kleiner ist als der zweite Durchmesser (112, 312).

6. Injektor (800) nach Anspruch 5, worin der dritte Abschnitt (114, 714) zylindrisch oder konisch ist und sich distal verjüngt.

7. Injektor (800) nach Anspruch 5, worin der erste Abschnitt (106, 306, 706), der zweite Abschnitt (110, 110', 301, 710) und der dritte Abschnitt (114, 714) entlang einer Längsachse der Kolbenstange (100, 300, 700) konzentrisch angeordnet sind.

8. Injektor (800) nach Anspruch 5, worin eine Länge des zweiten Abschnitts (110, 110', 301, 710) entlang einer Längsachse zumindest zweimal so viel ist wie eine Länge des dritten Abschnitts (114, 714) entlang der Längsachse.

9. Injektor (800) nach Anspruch 1, worin das distale Ende (104, 304, 704) mit der Kolbenstange (100, 300, 700) oder dem Kolbenstangenadapter (728) einstückig ausgebildet ist.

10. Injektor (800) nach Anspruch 1, worin:
der Injektor (800) die Kolbenstange (100, 300, 700) und den Kolbenstangenadapter (728) umfasst, und
das distale Ende (104, 304, 704) der Kolbenstangenadapter (728) ist und mit der Kolbenstange (100, 300, 700) abnehmbar verbunden ist.

## Revendications

1. Injecteur (800) pour injecter un médicament, l'injecteur (800) comprenant :
un corps de seringue (202) ;
un piston (200, 400) disposé de manière mobile dans le corps de seringue (202), le piston (200, 400) définissant un espace intérieur (206, 406) s'étendant depuis une face proximale (218, 418) du piston (200, 400) jusqu'à une portion distale (212, 412), le piston (200, 400) comprenant des filets (204, 404) comprenant des sommets (220, 420) ;
une tige de piston (100, 300, 700) ou un adaptateur de tige de piston (728) pour déplacer axialement le piston (200, 400) dans le corps de seringue (202), la tige de piston (100, 300, 700) ou l'adaptateur de tige de piston (728) comprenant :
une extrémité proximale (102) ; et
une extrémité distale (104, 304, 704) comprenant :
une première portion (106, 306, 706) présentant un premier diamètre (108, 308, 708) ; et
une deuxième portion (110, 110', 301, 710) distale par rapport à la première portion (106, 306, 706), la deuxième portion (110, 110', 301, 710) présentant un deuxième diamètre (112, 312) inférieur au premier diamètre (108, 308, 708), dans lequel
la deuxième portion (110, 110', 301, 710) présente une géométrie extérieure qui est complémentaire de la portion distale (212, 412) de l'espace intérieur (206, 406) du piston (200, 400), et
l'extrémité distale (104, 304, 704) est configurée de telle sorte que lorsque la tige de piston (100, 300, 700) ou l'adaptateur de tige de piston (728) est inséré par coulissement dans l'espace intérieur du piston (200, 400) :
la première portion (106, 306, 706) vient en contact avec la face proximale du piston (200, 400), et **caractérisé en ce que**
la deuxième portion (110, 110', 301, 710) vient en appui contre chacun des sommets (220, 420) des filets (204, 404) de l'espace intérieur (206, 406) du piston (200, 400) sans être reliée de manière amovible au piston (200, 400) de telle sorte que la tige de piston (100, 300, 700) ou l'adaptateur de tige de piston (728) est configuré pour se dégager du piston (200, 400) par coulissement de la tige de piston (100, 300, 700) ou de l'adaptateur de tige de piston (728) en direction proximale à l'écart du piston (200, 400).

2. Injecteur (800) selon la revendication 1, dans lequel la géométrie extérieure est configurée pour venir en prise avec le piston (200, 400) de telle sorte que la deuxième portion (110, 110', 301, 710) est configurée pour résister à des forces de déformation appliquées au piston (200, 400) lors d'un déplacement axial du piston (200, 400) dans le corps de seringue (202).

3. Injecteur (800) selon la revendication 1, dans lequel la deuxième portion (110, 110', 301, 710) est conique et se rétrécit en direction distale.

4. Injecteur (800) selon la revendication 1, dans lequel l'extrémité distale comprend en outre une tête qui est configurée pour venir en contact avec la portion distale (212, 412) de l'espace intérieur (206, 406) du piston (200, 400).

5. Injecteur (800) selon la revendication 4, dans lequel l'extrémité distale (104, 304, 704) comprend en outre une troisième portion (114, 714) entre la deuxième portion (110, 110', 301, 710) et la tête, la troisième portion (114, 714) présente un troisième diamètre (116) qui est inférieur au deuxième diamètre (112, 312).

6. Injecteur (800) selon la revendication 5, dans lequel la troisième portion (114, 714) est cylindrique ou est conique et se rétrécit en direction distale.

7. Injecteur (800) selon la revendication 5, dans lequel la première portion (106, 306, 706), la deuxième portion (110, 110', 301, 710) et la troisième portion (114, 714) sont agencées de manière concentrique le long d'un axe longitudinal de la tige de piston (100, 300, 700).

8. Injecteur (800) selon la revendication 5, dans lequel une longueur de la deuxième portion (110, 110', 301, 710) le long d'un axe longitudinal est au moins le double d'une longueur de la troisième portion (114, 714) le long de l'axe longitudinal.

9. Injecteur (800) selon la revendication 1, dans lequel l'extrémité distale (104, 304, 704) est venue de matière avec la tige de piston (100, 300, 700) ou l'adaptateur de tige de piston (728).

10. Injecteur (800) selon la revendication 1, dans lequel :
l'injecteur (800) comprend la tige de piston (100, 300, 700) et l'adaptateur de tige de piston (728), et
l'extrémité distale (104, 304, 704) est l'adaptateur de tige de piston (728) et est reliée de manière amovible à la tige de piston (100, 300, 700).
